# EUROPEAN PATENT APPLICATION

(11) **EP 4 799 639 A1**
(43) Date of publication of application: **02.09.2026**
(21) Application number: 23961604.8
(22) Date of filing: 14.12.2023
(51) Int. Cl.: A61K 41/00, A61K 47/54, A61P 35/00, A61P 37/02

(54) **ANTICANCER COMPOSITION COMPRISING METAL-ORGANIC FRAMEWORK HAVING IMMUNO-ANTICANCER AGENT BONDED THERETO, AND USE THEREOF**

(30) Priority: 12.12.2023 KR 20230179991
(71) Applicant: Mediark Inc., Seowon-gu Cheongju-si, Chungcheongbuk-do 28644 (KR)
(72) Inventor: KIM, Se Na, Cheongju-si, Chungcheongbuk-do, 28181 (KR); CHUN, Nea Young, Yongin-si, Gyeonggi-do 16862 (KR); OH, Yu Jin, Sejong-si 30150 (KR)
(74) Representative: BCKIP Part mbB
(86) International application number: PCT/KR2023/020618
(87) International publication number: WO 2025/127203

(57) **Abstract**

The present invention relates to an anticancer composition comprising a metal-organic framework having a cancer immunotherapeutic agent linked thereto and the use thereof. The anticancer composition is capable of sustained release of the cancer immunotherapeutic agent linked to the metal-organic framework and may increase the efficiency of delivery to a site where cancer cells rather than normal cells are located, and the metal-organic framework is capable of exhibiting a photodynamic therapeutic effect of killing cancer cells by generating singlet oxygen upon light irradiation. The present invention also provides a method for treating cancer, comprising a step of administering the anticancer composition to a subject and then irradiating the subject with light.

## Description

### Technical Field

The present invention relates to an anticancer composition comprising a metal-organic framework having a cancer immunotherapeutic agent linked thereto and the use thereof.

### Background Art

Cancer is one of the incurable diseases that humans have to overcome, and huge amounts of capital have been invested in development to cure cancer worldwide. In Korea, cancer is the number one cause of death by disease, with more than 100,000 people diagnosed with cancer each year and more than 60,000 people dying from cancer.

Common anticancer therapies for cancer treatment include surgery, chemotherapy, and radiotherapy. Thereamong, patients who are not easily treated with surgery or radiotherapy (approximately 50% of all cancer cases) and patients whose cancer has already metastasized are mainly treated with chemotherapy. However, due to drug resistance, recurrence, metastasis, and aftereffects, the development of cancer treatment technologies that can minimize side effects has recently become important.

Cancer immunotherapeutic agents, called third-generation cancer treatments, work by activating the body's immune system to induce immune cells to attack cancer cells. Unlike firstgeneration chemotherapeutic agents or second-generation targeted anticancer drugs, cancer immunotherapeutic agents are expected to reduce the side effects of anticancer drugs and exhibit maximized therapeutic effects.

However, even in the case of third-generation cancer immunotherapeutic agents, when the immune system is excessively activated due to the cancer immunotherapeutic agent, side effects such as immune disease symptoms in which immune cells attack not only cancer cells but also normal cells occur.

There is a need for the development of therapeutic agents that overcome the above-described problems and, at the same time, can dramatically improve patient safety by minimizing systemic exposure of cancer immunotherapeutic agents and exposure to normal cells.

### [Prior Art Documents]

### [Patent Documents]

KR 10-2022-0151333 A1

### DISCLOSURE

### Technical Problem

An object of the present invention is to provide an anticancer composition comprising a metal-organic framework having a cancer immunotherapeutic agent linked thereto and the use thereof.

Another object of the present invention is to provide an anticancer composition comprising a metal-organic framework having a cancer immunotherapeutic agent linked thereto, wherein the anticancer composition is capable of sustained release of the cancer immunotherapeutic agent linked to the metal-organic framework and may increase the efficiency of delivery to a site where cancer cells rather than normal cells are located, and wherein the metal-organic framework is capable of exhibiting a photodynamic therapeutic effect of killing cancer cells by generating singlet oxygen upon light irradiation.

Still another object of the present invention is to provide a method for treating cancer, comprising a step of administering the anticancer composition to a subject and then irradiating the subject with light.

### Technical Solution

To achieve the above objects, the present invention provides an anticancer composition comprising a metal-organic framework (MOF) having a cancer immunotherapeutic agent linked thereto.

In addition, the metal-organic framework may comprise a metal cluster and a ligand compound represented by Formula 1 below that coordinates to the metal cluster:

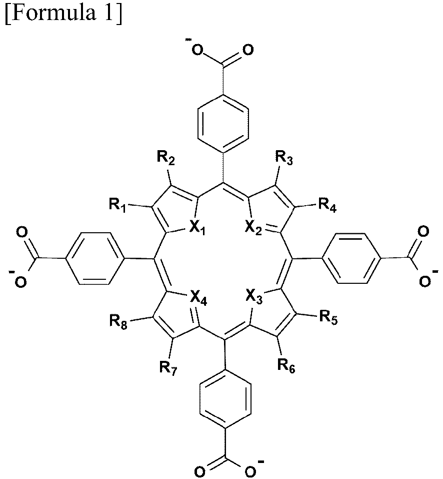

wherein
X₁ and X₃ are each independently N(R₉),
X₂ and X₄ are each independently N, and
R₁ to R₉ are the same as or different from one another and are each independently selected from the group consisting of hydrogen, deuterium, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

In addition, the metal-organic framework may comprise either a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir³⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ and Bi⁺.

In addition, the metal-organic framework may be selected from the group consisting of an aluminum-based metal-organic framework, an iron-based metal-organic framework, a zirconium-based metal-organic framework, and mixtures thereof.

In addition, the cancer immunotherapeutic agent may coordinate to an unsaturated metal site of the metal cluster.

In addition, the cancer immunotherapeutic agent may be an immune-modulating agent.

In addition, the immune-modulating agent may be an agonist of TLR7 (Toll-like receptor 7) or TLR8.

In addition, the cancer may be colorectal cancer, liver cancer, lung cancer, breast cancer, melanoma, gastric cancer, colon cancer, skin cancer, ovarian cancer, cervical cancer, thyroid cancer, kidney cancer, prostate cancer, bladder cancer, pancreatic cancer, esophageal cancer, or fibrosarcoma.

In addition, the metal-organic framework may generate singlet oxygen upon light irradiation.

A method for treating cancer according to another embodiment of the present invention may comprise a step of administering the anticancer composition to a subject in an amount effective for treating the cancer and irradiating the subject with light.

In the present invention, the "metal-organic framework (MOF)" refers to a porous material in which a metal cluster and an organic linker (or an organic bridging ligand) are linked to each other by coordination to form a three-dimensional structure. Various MOFs may be produced depending on the choice of the metal ion and the organic ligand. The MOF has a porous characteristic in which voids exist in the structure, and the pore size, porosity, three-dimensional structure, surface area, etc. of the MOF may be designed in various ways depending on the types of metal ion and organic ligand constituting the MOF and a method for linking them. Due to their porous characteristic, MOFs not only have a very large surface area, but also have an open pore structure, and thus they allow large amounts of molecules or solvents to move therethrough compared to other porous materials known to date. In addition, when MOFs are used as catalysts or gas storage media, they may advantageously maximize efficiency due to many active sites present therein. In addition, the MOFs do not easily deform at high temperatures and have a rigid skeleton, and thus they have excellent chemical and thermal stability.

In the present invention, the "subject" may include, without limitation, mammals, including dogs, cats, rats, livestock, humans, etc., birds, reptiles, farmed fish, etc., and the subject may exclude humans.

In the present invention, unless specifically stated otherwise, "hydrogen" is hydrogen, light hydrogen, deuterium or tritium.

In the present invention, "halogen group" is fluorine, chlorine, bromine or iodine.

In the present invention, "alkyl" refers to a monovalent substituent derived from a linear or branched saturated hydrocarbon having 1 to 40 carbon atoms. Examples thereof include, but are not limited to, methyl, ethyl, propyl, isobutyl, sec-butyl, pentyl, iso-amyl, hexyl, and the like.

In the present invention, "alkenyl" refers to a monovalent substituent derived from a linear or branched unsaturated hydrocarbon having 2 to 40 carbon atoms and at least one carbon-carbon double bond. Examples thereof include, but are not limited to, vinyl, allyl, isopropenyl, 2-butenyl, and the like.

In the present invention, "alkynyl" refers to a monovalent substituent derived from a linear or branched unsaturated hydrocarbon having 2 to 40 carbon atoms and at least one carbon-carbon triple bond. Examples thereof include, but are not limited to, ethynyl, 2-propynyl, and the like.

In the present invention, "alkylthio" refers to the above-described alkyl group attached via a sulfur linkage (-S-).

In the present invention, "aryl" refers to a monovalent substituent derived from an aromatic hydrocarbon having 6 to 60 carbon atoms with a single ring or a combination of two or more rings. In addition, a form in which two or more rings are simply pendant to each other (pendant) or fused together may also be included. Specific examples of the aryl include, but are not limited to, a naphthyl group, an anthracenyl group, a phenanthryl group, a triphenyl group, a pyrenyl group, a phenalenyl group, a perylenyl group, a chrysenyl group, a fluorenyl group, etc. The fluorenyl group may be substituted, and adjacent groups may be bonded to each other to form a ring.

In the present invention, "heteroaryl" refers to a monovalent substituent derived from a monoheterocyclic or polyheterocyclic aromatic hydrocarbon having 6 to 30 carbon atoms. Here, at least one, preferably 1 to 3 carbon atoms, in the ring, is substituted with a heteroatom such as N, O, S or Se. In addition, a form in which two or more rings are simply pendant to each other or fused together may be included, and further, a form in which two or more rings are fused to an aryl group may also be included. Examples of the heteroaryls include, but are not limited to, 6-membered monocyclic rings such as pyridyl, pyrazinyl, pyrimidinyl, pyridazinyl, and triazinyl; polycyclic rings such as phenoxathienyl, indolizinyl, indolyl, purinyl, quinolyl, benzothiazole, and carbazolyl; and 2-furanyl, N-imidazolyl, 2-isoxazolyl, 2-pyridinyl, 2-pyrimidinyl, etc.

In the present invention, "aryloxy" refers to a monovalent substituent represented by RO-, wherein R represents an aryl having 6 to 60 carbon atoms. Examples of the aryloxy include, but are not limited to, phenyloxy, naphthyloxy, diphenyloxy, and the like.

In the present invention, "alkyloxy" refers to a monovalent substituent represented by R'O-, wherein R' represents an alkyl having 1 to 40 carbon atoms. The alkyloxy may include a linear, branched, or cyclic structure. Examples of the alkyloxy include, but are not limited to, methoxy, ethoxy, n-propoxy, 1-propoxy, t-butoxy, n-butoxy, pentoxy, and the like.

In the present invention, "alkoxy" may be linear, branched or cyclic. The number of carbon atoms of the alkoxy is not particularly limited, but is preferably 1 to 20. Specific examples of the alkoxy include, but are not limited to, methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like.

In the present invention, "aralkyl" refers to an aryl-alkyl group wherein aryl and alkyl are as defined above. Preferred aralkyl groups include lower alkyl groups. Non-limiting examples of suitable aralkyl groups include benzyl, 2-phenethyl, and naphthalenylmethyl. Bonding to the parent moiety is via the alkyl.

In the present invention, "arylamino group" refers to an amine substituted with an aryl group having 6 to 30 carbon atoms.

In the present invention, "alkylamino group" refers to an amine substituted with an alkyl group having 1 to 30 carbon atoms.

In the present invention, "aralkylamino group" refers to an amine substituted with an aryl-alkyl group having 6 to 30 carbon atoms.

In the present invention, "heteroarylamino group" refers to an amine group substituted with an aryl group having 6 to 30 carbon atoms and a heterocyclic group.

In the present invention, "heteroaralkyl group" refers to an aryl-alkyl group substituted with a heterocyclic group.

In the present invention, "cycloalkyl" refers to a monovalent substituent derived from a monocyclic or polycyclic non-aromatic hydrocarbon having 3 to 40 carbon atoms. Examples of the cycloalkyl include, but are not limited to, cyclopropyl, cyclobutyl, cyclopentyl, cyclohexyl, norbornyl, adamantine, and the like.

In the present invention, "heterocycloalkyl" refers to a monovalent substituent derived from a non-aromatic hydrocarbon having 3 to 40 carbon atoms, wherein at least one, preferably 1 to 3 carbon atoms, in the ring, is substituted with a heteroatom such as N, O, S or Se. Examples of the heterocycloalkyl include, but are not limited to, morpholine, piperazine, and the like.

In the present invention, "alkylsilyl" refers to silyl substituted with an alkyl having 1 to 40 carbon atoms, and "arylsilyl" refers to silyl substituted with an aryl having 6 to 60 carbon atoms.

In the present invention, "fused ring" refers to a fused aliphatic ring, a fused aromatic ring, a fused heteroaliphatic ring, a fused heteroaromatic ring, or a combination thereof.

In the present invention, "adjacent groups may be bonded to each other to form a ring" means that adjacent groups are bonded to each other to form a substituted or unsubstituted aliphatic hydrocarbon ring; a substituted or unsubstituted aromatic hydrocarbon ring; a substituted or unsubstituted aliphatic heterocyclic ring; a substituted or unsubstituted aromatic heterocyclic ring; or a fused ring thereof.

Examples of the "aromatic hydrocarbon ring" in the present invention include, but are not limited to, a phenyl group, a naphthyl group, an anthracenyl group, etc.

In the present invention, "aliphatic heterocyclic ring" refers to an aliphatic ring containing at least one heteroatom.

In the present invention, "aromatic heterocyclic ring" refers to an aromatic ring containing at least one heteroatom.

In the present invention, "substitution" means that a hydrogen atom bonded to a carbon atom of a compound is replaced with another substituent, wherein a position to be substituted is not limited as long as it is a position where the hydrogen atom is substituted, that is, a position where the hydrogen atom may be substituted with a substituent, and when two or more hydrogen atoms are substituted, the two or more substituents may be the same as or different from each other. The substituent may be at least one substituent selected from the group consisting of hydrogen, a cyano group, a nitro group, a halogen group, a hydroxy group, an alkyl group having 1 to 30 carbon atoms, an alkenyl group having 2 to 30 carbon atoms, an alkynyl group having 2 to 24 carbon atoms, a heteroalkyl group having 2 to 30 carbon atoms, an aralkyl group having 6 to 30 carbon atoms, an aryl group having 5 to 30 carbon atoms, a heteroaryl group having 2 to 30 carbon atoms, a heteroarylalkyl group having 3 to 30 carbon atoms, an alkoxy group having 1 to 30 carbon atoms, an alkylamino group having 1 to 30 carbon atoms, an arylamino group having 6 to 30 carbon atoms, an aralkylamino group having 6 to 30 carbon atoms, a heteroarylamino group having 2 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms, without being limited thereto.

### Advantageous Effects

The present invention provides an anticancer composition comprising a metal-organic framework having a cancer immunotherapeutic agent linked thereto, wherein the anticancer composition is capable of sustained release of the cancer immunotherapeutic agent linked to the metal-organic framework and may increase the efficiency of delivery to a site where cancer cells rather than normal cells are located, and wherein the metal-organic framework is capable of exhibiting a photodynamic therapeutic effect of killing cancer cells by generating singlet oxygen upon light irradiation.

The present invention also provides a method for treating cancer, comprising a step of administering the anticancer composition to a subject and then irradiating the subject with light.

### Brief Description of Drawings

FIG. 1 is a front view of the structure of a metal-organic framework according to one embodiment of the present invention.
FIG. 2 is a side view of the structure of a metal-organic framework according to one embodiment of the present invention.
FIG. 3 is a scanning electron microscopy image of a metal-organic framework according to one embodiment of the present invention.
FIG. 4 shows the results of X-ray diffraction analysis of a metal-organic framework according to one embodiment of the present invention.
FIG. 5 shows the results of measuring the N₂ adsorption/desorption isotherm, specific surface area, and pore diameter of a metal-organic framework according to one embodiment of the present invention.
FIG. 6 shows the results of measuring the zeta potential of a metal-organic framework according to one embodiment of the present invention.
FIG. 7 shows the results of FT-IR analysis of a metal-organic framework according to one embodiment of the present invention.
FIG. 8 is a front view of the structure of a metal-organic framework according to one embodiment of the present invention.
FIG. 9 is a side view of the structure of a metal-organic framework according to another embodiment of the present invention.
FIG. 10 is a scanning electron microscopy image of a metal-organic framework according to one embodiment of the present invention.
FIG. 11 shows the results of X-ray diffraction analysis of a metal-organic framework according to one embodiment of the present invention.
FIG. 12 shows the results of measuring the N₂ adsorption/desorption isotherm, specific surface area, and pore diameter of a metal-organic framework according to one embodiment of the present invention.
FIG. 13 shows the results of measuring the zeta potential of a metal-organic framework according to one embodiment of the present invention.
FIG. 14 shows the results of FT-IR analysis of a metal-organic framework according to one embodiment of the present invention.
FIG. 15 is a front view of the structure of a metal-organic framework according to one embodiment of the present invention.
FIG. 16 is a side view of the structure of a metal-organic framework according to one embodiment of the present invention.
FIG. 17 is a scanning electron microscopy image of a metal-organic framework according to one embodiment of the present invention.
FIG. 18 shows the results of X-ray diffraction analysis of a metal-organic framework according to one embodiment of the present invention.
FIG. 19 shows the results of measuring the N₂ adsorption/desorption isotherm, specific surface area, and pore diameter of a metal-organic framework according to one embodiment of the present invention.
FIG. 20 shows the results of measuring the zeta potential of a metal-organic framework according to one embodiment of the present invention.
FIG. 21 shows the results of FT-IR analysis of a metal-organic framework according to one embodiment of the present invention.
FIG. 22 shows the results of a cytotoxicity test for a metal-organic framework according to one embodiment of the present invention.
FIG. 23 shows the results of a cytotoxicity test for a metal-organic framework according to one embodiment of the present invention.
FIG. 24 shows the results of a cytotoxicity test for a metal-organic framework according to one embodiment of the present invention.
FIG. 25 shows the results of conducting a photodynamic therapy (PDT) test using a metal-organic framework according to one embodiment of the present invention by light irradiation at different laser power levels for 5 minutes.
FIG. 26 shows the results of conducting a PDT test using a metal-organic framework according to one embodiment of the present invention by light irradiation for a cumulative time of 3 minutes.
FIG. 27 shows the results of conducting a PDT test using a metal-organic framework according to one embodiment of the present invention by light irradiation for a cumulative time of 10 minutes.
FIG. 28 shows the results of testing the cytotoxicity of a metal-organic framework according to one embodiment of the present invention against colorectal cancer cells.
FIG. 29 shows scanning electron microscopy images of metal-organic frameworks having a cancer immunotherapeutic agent linked thereto according to one embodiment of the present invention.
FIG. 30 shows the results of conducting a PDT test using metal-organic frameworks having a cancer immunotherapeutic agent linked thereto according to one embodiment of the present invention by light irradiation for a cumulative time of 10 minutes.
FIG. 31 shows the results of testing the cytotoxicity of metal-organic frameworks having a cancer immunotherapeutic agent linked thereto according to one embodiment of the present invention against colorectal cancer cells.
FIG. 32 shows the results of a cytotoxicity test for a metal-organic framework having a cancer immunotherapeutic agent linked thereto according to one embodiment of the present invention.
FIG. 33 shows the results of a cytotoxicity test for a metal-organic framework having a cancer immunotherapeutic agent linked thereto according to one embodiment of the present invention.
FIG. 34 shows the results of a cytotoxicity test for a metal-organic framework having a cancer immunotherapeutic agent linked thereto according to one embodiment of the present invention.

### Best Mode

The present invention relates to an anticancer composition comprising a metal-organic framework (MOF) having a cancer immunotherapeutic agent linked thereto.

### Mode for Invention

Hereinafter, embodiments of the present invention will be described in detail so that those skilled in the art can easily carry out the present invention. However, the present invention may be embodied in various different forms and is not limited to the embodiments described herein.

Recently, light-based therapy has been gaining attention as a new cancer treatment technology that is non-invasive and has minimal side effects. This therapy, called phototherapy, may be divided into photothermal therapy and photodynamic therapy.

The photothermal therapy is a technology that treats cancer by heat generated when a light-absorbing substance is irradiated with light of a specific wavelength, and the photodynamic therapy is a technology that kills cancer by a secondary chemical reaction occurring when light is absorbed.

More specifically, the photodynamic therapy is performed based on the principle that, when a photosensitizer is activated by light of a specific wavelength, it interacts with the oxygen present in the tissue to produce singlet oxygen (¹O₂), which necrotizes surrounding cell molecules by strong oxidative ability. Reactive oxygen species, such as singlet oxygen, when present in small amounts, are essential for various physiological functions such as muscle contraction, fat metabolism, stem cell differentiation, and cell maintenance, but when overexpressed, they can cause cell death, carcinogenesis, tissue senescence, and the like.

In particular, the photodynamic therapy has great advantages in that it is noninvasive, allows repeated treatment, making it easy to increase its effectiveness, and may be used in combination with other therapies such as anticancer chemotherapy, and thus it may also be used as adjuvant treatment.

Accordingly, the present invention relates to an anticancer composition characterized by comprising a metal-organic framework capable of photodynamic therapy and a cancer immunotherapeutic agent.

That is, as described above, the photodynamic therapy may be used as an adjuvant therapy that may be used in combination with other anticancer therapies, and thus the present invention relates to a composition that is capable of performing photodynamic therapy using a metal-organic framework as well as anticancer treatment by a cancer immunotherapeutic agent.

The anticancer composition of the present invention may comprise a metal-organic framework (MOF) having a cancer immunotherapeutic agent linked thereto. Specifically, the composition is characterized in that the cancer immunotherapeutic agent is linked to the metal-organic framework.

As described above, the cancer immunotherapeutic agent works by activating the body's immune system to induce immune cells to attack cancer cells, and is expected to reduce the side effects of anticancer drugs and maximize the therapeutic effects. However, when the immune system is excessively activated due to the cancer immunotherapeutic agent, side effects such as immune disease symptoms in which immune cells attack not only cancer cells but also normal cells occur.

In order to overcome the above-described problems, according to the present invention, as the cancer immunotherapeutic agent is linked to the metal-organic framework, the cancer immunotherapeutic agent can be sustainedly released by a single administration, and an excessive amount of the cancer immunotherapeutic agent is not exposed systemically, but can be released at the site, where cancer cells are located, by the metal-organic framework, thereby overcoming the problems of conventional cancer immunotherapeutic agents.

The cancer immunotherapeutic agent may be an immune-modulating agent. Here, immune-modulating agent may be an agonist of TLR7 (Toll-like receptor 7) or TLR8.

The Toll-like receptor (TLR) is mainly expressed in immune cells and plays an important role in the natural immune response of mammals. TLR detects pathogen-associated molecular patterns (PAMP) and stimulates immune cells through the MyD88-dependent interleukin 1 receptor (IL-1R)-TLR signaling pathway, resulting in activation of the transcription factor NF-κB2. Ten functional TLR family members (TLR1 to TLR10) have been identified in humans (Akira S. et al., Nature Immunol., (2001) 2:675-680). TLR targets undergoing clinical trials for application in cancer immunotherapy include TLR3, 4, 7, 8, and 9 (SE Lee and JH Rhee, J. Bacteriol. Virol. (2012), 42(3):255-262).

The cancer immunotherapeutic agent may be an imidazoquinoline compound, more specifically resiquimod (R-848), without being limited thereto.

Resiquimod (R-848) is a drug that acts as an immune response modifier and has antiviral and antitumor effects. It may be used as a topical gel in the treatment of skin lesions such as those caused by herpes simplex virus and cutaneous T cell lymphoma and, as an adjuvant to increase the effectiveness of vaccines. In an animal disease model, systemic administration of resiquimod-loaded nanoparticles has been shown to improve response rates to cancer immunotherapy with a checkpoint inhibitor through stimulation of tumor-associated macrophages. It has several mechanisms of action, including an agonist of Toll-like receptor 7 (TLR7) and TLR8, and an upregulator of the opioid growth factor receptor.

The cancer immunotherapeutic agent may coordinate to the unsaturated metal site of the metal cluster.

The metal-organic framework of the present invention may comprise a metal cluster and a ligand compound represented by Formula 1 below that coordinates to the metal cluster: 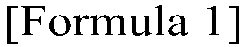 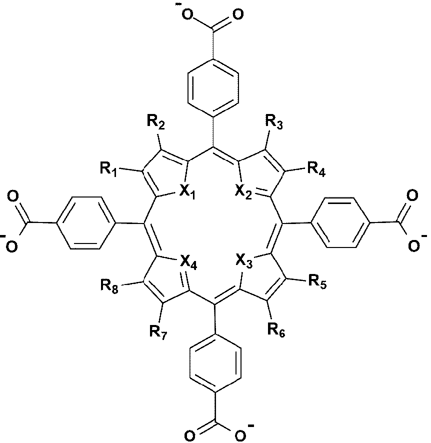 wherein
X₁ and X₃ are each independently N(R₉),
X₂ and X₄ are each independently N, and
R₁ to R₉ are the same as or different from one another and are each independently selected from the group consisting of hydrogen, deuterium, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

The metal cluster contained in the metal-organic framework contains an unsaturated metal site capable of forming a coordination bond. The cancer immunotherapeutic agent may be linked by coordination to the unsaturated metal site. The metal-organic framework having the cancer immunotherapeutic agent linked thereto may be configured to be actively degraded by irradiating the site where release of the cancer immunotherapeutic agent is required with light, thereby releasing the loaded cancer immunotherapeutic agent, thus enabling release at the target site (a specific region), and at the same time, may be configured to be capable of sustained release by controlling the amount of light irradiation.

The metal-organic framework may comprise either a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir³⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ and Bi⁺.

The metal-organic framework may be selected from the group consisting of an aluminum-based metal-organic framework, an iron-based metal-organic framework, a zirconium-based metal-organic framework, and mixtures thereof.

However, preferably, the metal-organic framework may be a metal-organic framework formed by linkage between the ligand represented by Formula 1 and a metal ion. More specifically, it may be PCN-221, PCN-222, PCN-223, PCN-224, MOF-545 or MOF-525, without being limited thereto.

The ligand may be a compound wherein R₁ to R₉ are all hydrogen. Specifically, it may be a compound represented by Formula 2 below:

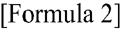

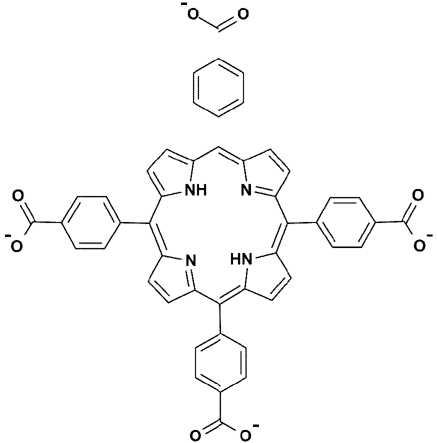

The -COO- group of the ligand compound represented by Formula 2 can form a coordination bond with the metal cluster to form the metal-organic framework.

As described above, the metal-organic framework of the present invention may exhibit not only the effect of releasing the linked cancer immunotherapeutic agent by being actively decomposed through irradiation of the target site with light, but also a photodynamic therapeutic effect of killing cancer cells using singlet oxygen generated by the metal-organic framework upon light irradiation.

The photodynamic therapy is a tripartite non-toxic process that requires the simultaneous presence of a photosensitizer (PS), light, and molecular oxygen. Although each component is individually harmless, when the photosensitizer is activated by light, energy is transferred to molecular oxygen, which generates highly cytotoxic reactive oxygen species (ROS). Among these ROS, singlet oxygen (¹O₂) can damage tumor cells. The photosensitizer is an important component in effective photodynamic therapy.

The ligand compound represented by Formula 1 is a very effective photosensitizer.

In addition, the metal-organic framework having a porous structure as disclosed in the present invention can integrate the photosensitizer into a periodic array exhibiting a high photosensitizer loading, and can provide a facile ¹O₂ diffusion path that improves the ¹O₂ generation efficiency. Thus, the metal-organic framework is an excellent photosensitizer.

The metal-organic framework of the present invention has high stability, a large surface area, and pores of an appropriate size for rapid oxygen diffusion, and is highly efficient as a photosensitizer.

As described above, the anticancer composition of the present invention is characterized by comprising the metal-organic framework having the cancer immunotherapeutic agent linked thereto.

The metal-organic framework can exhibit a photodynamic therapeutic effect by generating singlet oxygen upon light irradiation. In addition, it can be actively decomposed upon light irradiation, thereby exhibiting the effect of releasing the linked cancer immunotherapeutic agent at a specific site.

Due to these characteristics, conventional cancer immunotherapeutic agents have been problematic due to side effects resulting from systemic exposure, but according to the present invention, the cancer cell killing effect of the cancer immunotherapeutic agent may be enhanced by local exposure rather than systematic exposure by releasing the cancer immunotherapeutic agent by light irradiation only at the site where the cancer cells are located, and the anticancer effect can be further enhanced by photodynamic therapy using the metal-organic framework.

The cancer may be colorectal cancer, liver cancer, lung cancer, breast cancer, melanoma, gastric cancer, colon cancer, skin cancer, ovarian cancer, cervical cancer, thyroid cancer, kidney cancer, prostate cancer, bladder cancer, pancreatic cancer, esophageal cancer, or fibrosarcoma.

The anticancer composition of the present invention may be provided as an anticancer pharmaceutical composition.

The pharmaceutical composition of the present invention may be administered parenterally during clinical administration and may be used in the form of a general pharmaceutical preparation. Parenteral administration may refer to administration via routes other than oral, such as intrarectal, intravenous, intraperitoneal, intramuscular, intra-arterial, transdermal, intranasal, inhalation, intraocular, and subcutaneous. When formulation, commonly used diluents or excipients such as fillers, extenders, binders, wetting agents, disintegrating agents, and surfactants are used. Preparations for parenteral administration include sterile aqueous solutions, non-aqueous solutions, suspensions, emulsions, lyophilized preparations, and suppositories. As non-aqueous solvents and suspending agents, propylene glycol, polyethylene glycol, vegetable oils such as olive oil, and injectable esters such as ethyl oleate may be used. As a suppository base, witepsol, macrogol, Tween 61, cocoa butter, laurin butter, glycerogelatin, etc. may be used.

In addition, when the pharmaceutical composition of the present invention is used as a medicine, it may further contain one or more ingredients having the same or similar function, in addition to the active ingredient of the present invention. The component having the same or similar function may preferably be an anticancer drug. Here, the anticancer drug may comprise at least one selected from among actinomycin D, bleomycin sulfate, daunomycin, daunorubicin, doxorubicin, epirubicin, idarubicin, mitomycin, mitomycin-C, mitramycin, irinotecan, camptothecin, novobiocin, epirubicin, dactinomycin, amsacrine, teniposide, etoposide, cisplatin, carboplatin, oxaliplatin, paclitaxel, docetaxel, gefitinib, erlotinib afatinib, or pharmaceutically acceptable salts thereof and mixtures thereof, without being limited thereto.

In addition, the pharmaceutical composition may be used in admixture with various pharmaceutically acceptable carriers, such as saline or organic solvents, and may contain carbohydrates such as glucose, sucrose or dextran, antioxidants such as ascorbic acid or glutathione, chelating agents, low-molecular-weight proteins, or other stabilizers, in order to increase stability or absorbability.

A method for treating cancer according to another embodiment of the present invention may comprise a step of administering the composition to a subject in an amount effective for treating the cancer and irradiating the subject with light.

In the method for treating cancer according to the present invention, the composition comprises, as an active ingredient, the metal-organic framework which has the cancer immunotherapeutic agent linked thereto and may act as a photosensitizer to generate singlet oxygen. Accordingly, the composition is capable of exhibiting both a cancer immunotherapeutic effect and a photodynamic therapeutic effect, and thus may be used for treating cancer. The cancer immunotherapeutic agent and the metal-organic framework are as described above.

### Production Example 1

### Synthesis of PCN-223

200 mL of N,N-dimethylformamide (DMF) and 96 mg of tetrakis(4-carboxyphenyl)porphyrin (TCPP) were added and stirred, thus preparing a TCPP-containing solution. 54 mg of zirconyl chloride octahydrate (ZrOCl₂·8H₂O) was added to the TCPP-containing solution, followed by stirring.

Thereafter, the stirred mixture was allowed to react in an oven at 105°C for 17 hours. After the reaction, the reaction product was collected and centrifuged at 15,000 rpm for 10 minutes, and the supernatant was removed. The lower layer fraction was washed by resuspension in 30 ml of DMF. Next, the same washing process was performed three times, and the washed material was treated three times with 30 ml of ethanol. The upper layer of ethanol was discarded in the final centrifugation process, and then the lower layer of PCN-223 particles was dried in an oven at 80°C for 8 hours to obtain the PCN-223 particles. Powder X-ray diffraction (PXRD) analysis was performed to check whether the particles were properly produced.

### Synthesis of PCN-224

Particles were obtained in the same manner as PCN-223, except that the reaction was performed in an oven at 120°C for 24 hours.

### Synthesis of MOF-525

80 mL of N,N-dimethylformamide (DMF), 75 mg of tetrakis(4-carboxyphenyl)porphyrin (TCPP), and 1.75 g of benzoic acid were added and stirred, thus preparing a TCPP-containing solution. 220 mg of ZrOCl₂ was added to the TCPP-containing solution, followed by stirring.

Thereafter, the stirred mixture was allowed to react in an oven at 100°C for 24 hours. After the reaction, the reaction product was collected and centrifuged at 15,000 rpm for 10 minutes, and the supernatant was removed. The lower layer fraction was washed by resuspension in 30 ml of DMF. Next, the same washing process was performed three times, and the washed material was treated three times with 30 ml of ethanol. The upper layer of ethanol was discarded in the final centrifugation process, and then the lower layer of MOF-525 particles was dried in an oven at 80°C for 8 hours to obtain the MOF-525 particles. Powder X-ray diffraction (PXRD) analysis was performed to check whether the particles were properly produced.

### Experimental Example

### Analysis of Synthesis Results

For observation with a scanning electron microscope (SEM), 2 mg to 3 mg of a powder sample was placed in an Eppendorf tube, and 100 µl of ethanol was added thereto, followed by bath sonication. Thereafter, 10 µl of the solution was dropped on a silicon wafer and dried in an oven at 80°C. The silicon wafer with the dried sample was attached to a SEM mount using a carbon tape, and transferred to a SEM (Zeiss, model name: ULTRA PLUS) for imaging.

Next, for X-ray diffraction (XRD) analysis, the powder sample was carefully placed on the XRD sample holder, and the sample was pressed with an appropriate force using a glass slide. Next, all powder scattered around the sample holder was carefully removed, and the sample holder was transferred to the XRD instrument (Bruker, model name: D2 Phaser) and set up, and XRD analysis was performed in the range of 2°C to 30°C.

Next, for the measurement of the N₂ adsorption/desorption isotherm, BET specific surface area, and pore diameter, about 40 mg of a completely dried sample was prepared and carefully placed in a glass sampler using a glass funnel, and all powder accumulated around the glass tube, excluding the sample container at the bottom, was removed. Thereafter, an upper filter cap was attached and degassing was performed by vacuum heat treatment at 120°C for 12 hours in the pretreatment device. The weight of the pretreated sample was measured using a microbalance, and the weight of the empty cell that was previously measured was subtracted therefrom to determine the weight of the complete sample. The glass sampler was mounted on the N₂ adsorption-desorption instrument (MICROTAAC, model name: BELSORP MINI X) and measurements were conducted).

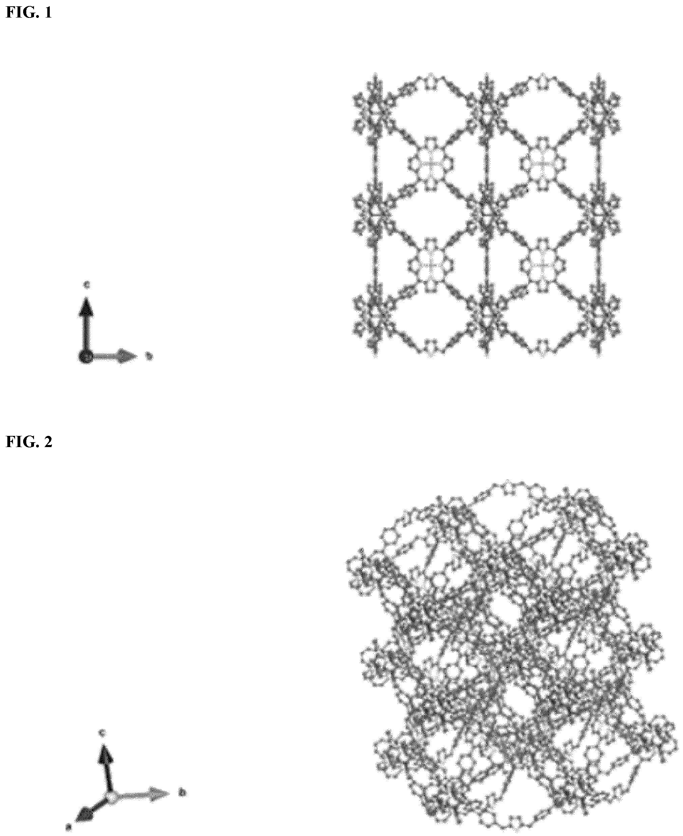

FIGS. 1 and 2 are images showing the structure of the PCN-223 particles.

FIG. 1 is an image showing the structure of PCN-223 viewed in the b direction (front), and FIG. 2 is an image showing the side structure of PCN-223.

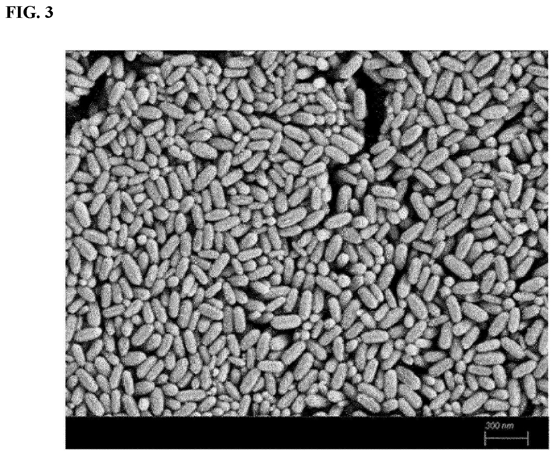

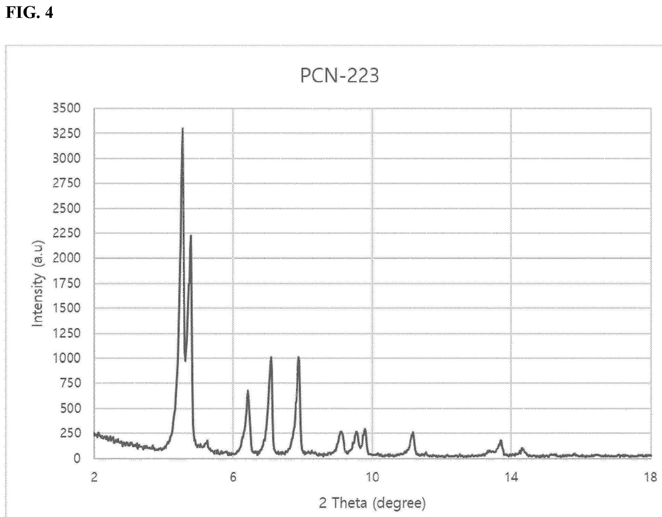

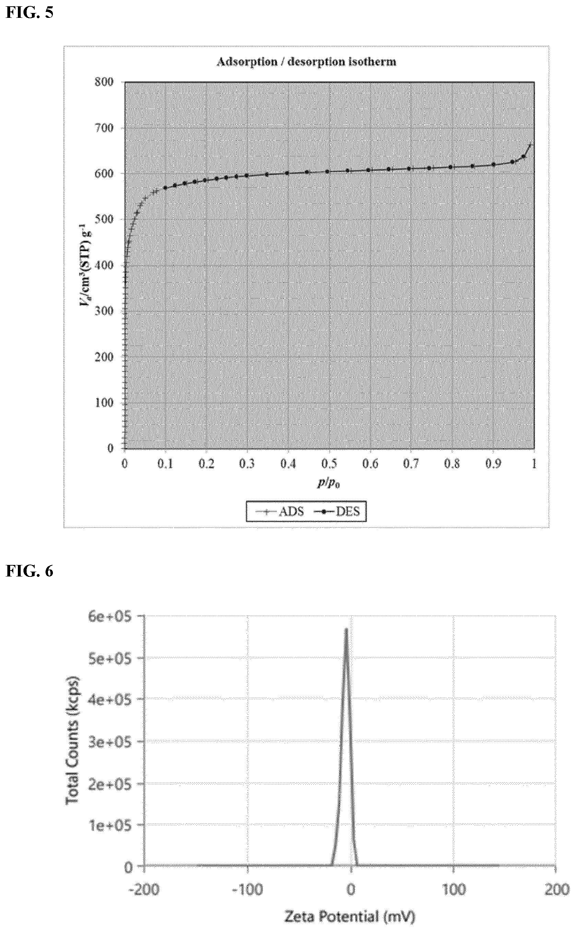

FIG. 3 is a scanning electron microscopy image of PCN-223. In addition, FIG. 4 shows the results of X-ray diffraction analysis of PCN-223. From these results, it was confirmed that the synthesized particles had the crystal structure of PCN-223. In addition, as a result of performing Brunauer-Emmett-Teller (BET) analysis through N₂ adsorption/desorption, it was confirmed that the PCN-223 particles had a specific surface area of 2,273.6 m²/g and a pore diameter of 1.42 nm (FIG. 5).

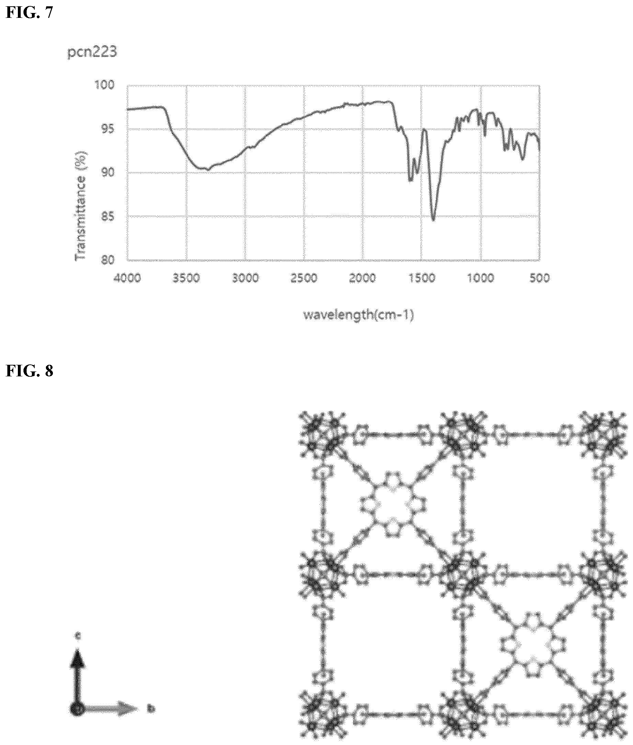

The zeta potential of PCN-223 was 4.26 mV (FIG. 6), and the results of FT-IR analysis of PCN-223 are shown in FIG. 7.

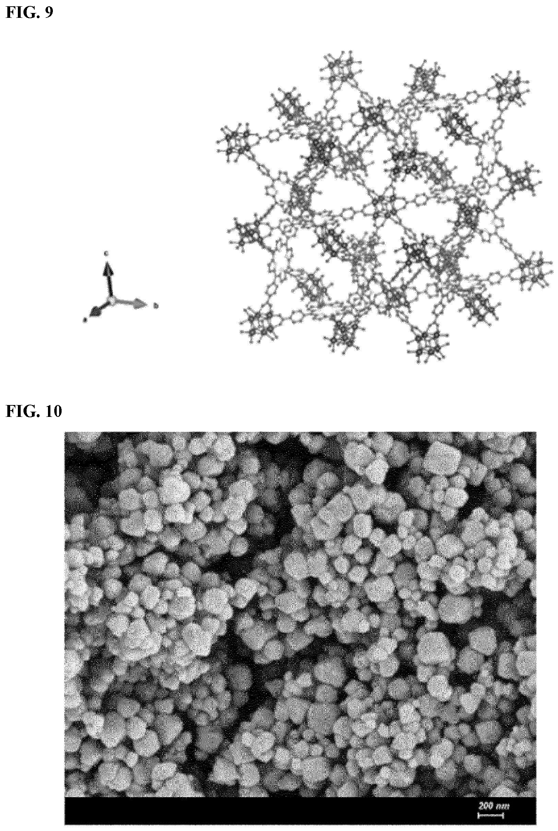

FIGS. 8 and 9 are images showing the structure of the PCN-224 particles. FIG. 8 is an image showing the structure of PCN-224 viewed in the b direction (front), and FIG. 9 is an image showing the side structure of PCN-224.

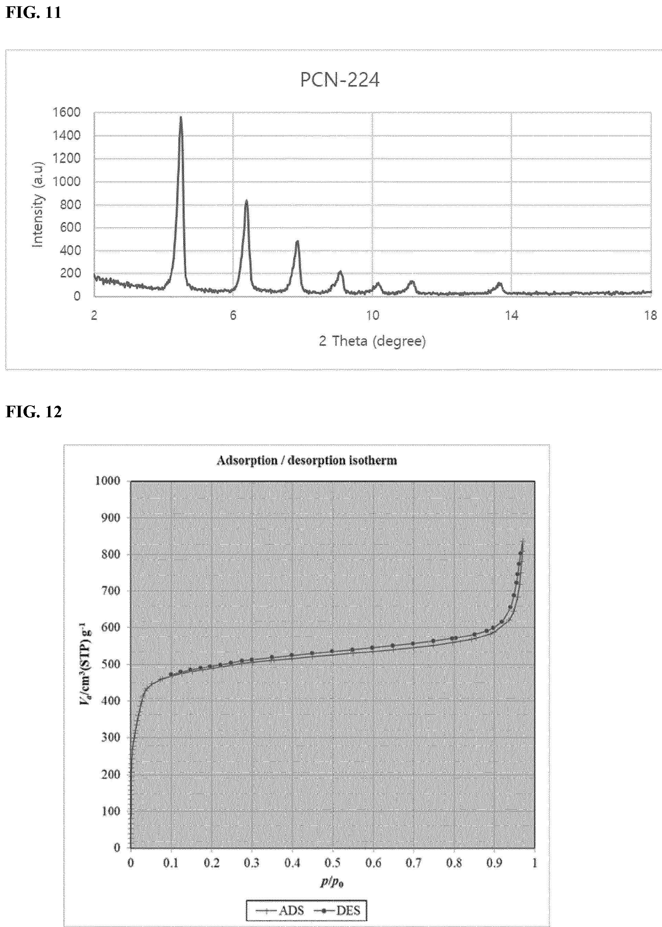

FIG. 10 is a scanning electron microscopy image of PCN-224. In addition, FIG. 11 shows the results of X-ray diffraction analysis of PCN-224. From these results, it was confirmed that the synthesized particles had the crystal structure of PCN-224. In addition, as a result of performing Brunauer-Emmett-Teller (BET) analysis through N₂ adsorption/desorption, it was confirmed that the PCN-224 particles had a specific surface area of 1,894 m²/g and a pore diameter of 1.61 nm (FIG. 12).

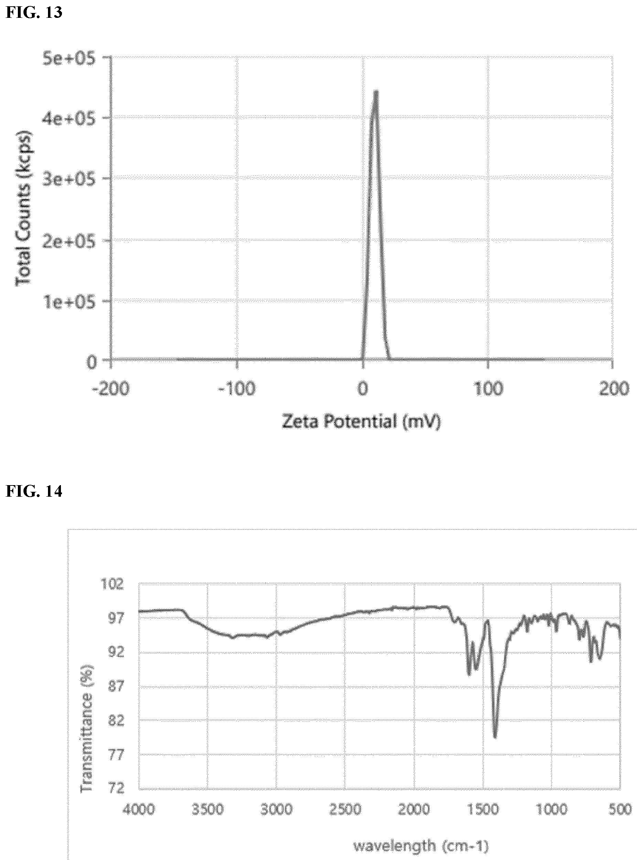

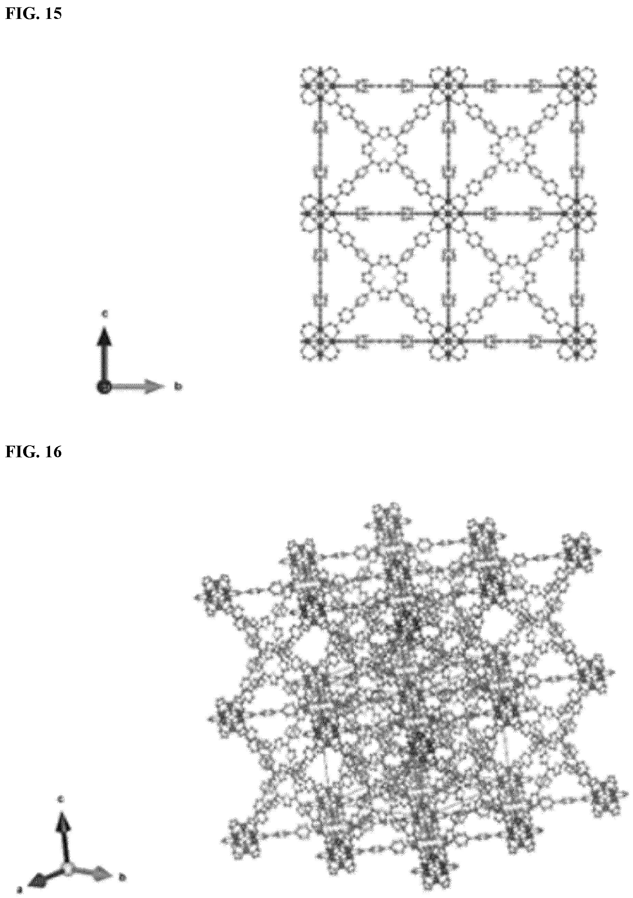

The zeta potential of PCN-224 was 10.43 mV (FIG. 13), and the results of FT-IR analysis of PCN-224 are shown in FIG. 14. FIGS. 15 and 16 are images showing the structure of the MOF-525 particles. FIG. 15 is an image showing the structure of MOF-525 viewed from the b direction (front), and FIG. 16 is an image showing the side structure of MOF-525.

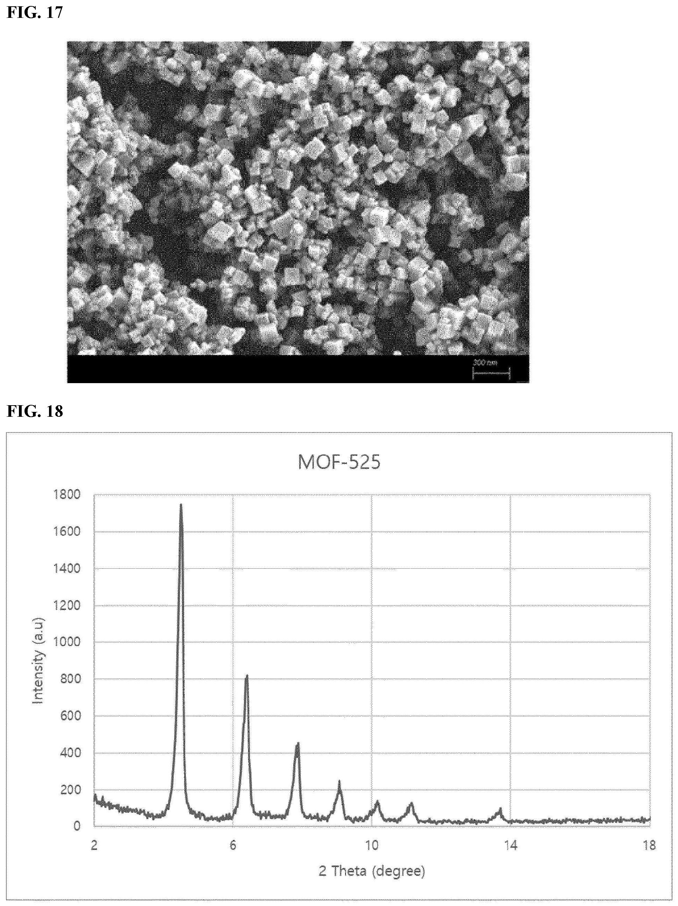

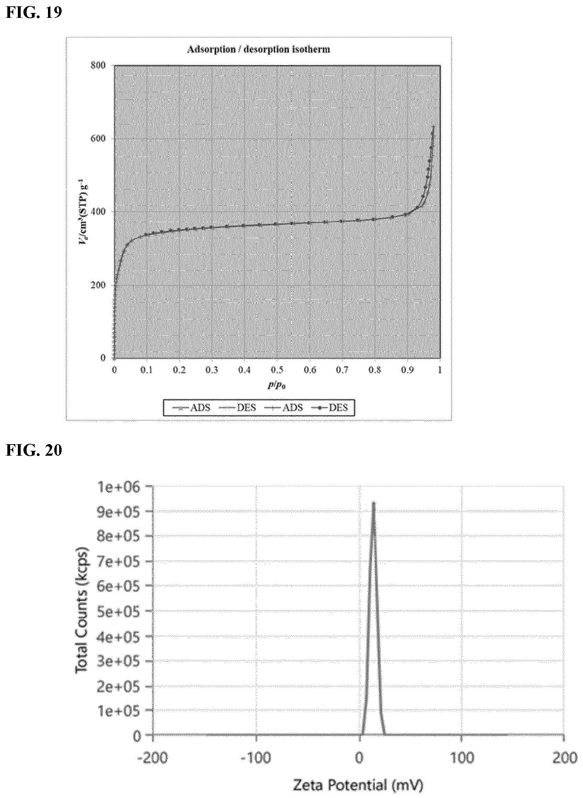

FIG. 17 is a scanning electron microscopy image of MOF-525. In addition, FIG. 18 shows the results of X-ray diffraction analysis of MOF-525. From these results, it was confirmed that the synthesized particles had the crystal structure of MOF-525. In addition, as a result of performing Brunauer-Emmett-Teller (BET) analysis through N₂ adsorption/desorption, it was confirmed that the MOF-525 particles had a specific surface area of 1,356.3 m²/g and a pore diameter of 1.68 nm (FIG. 19).

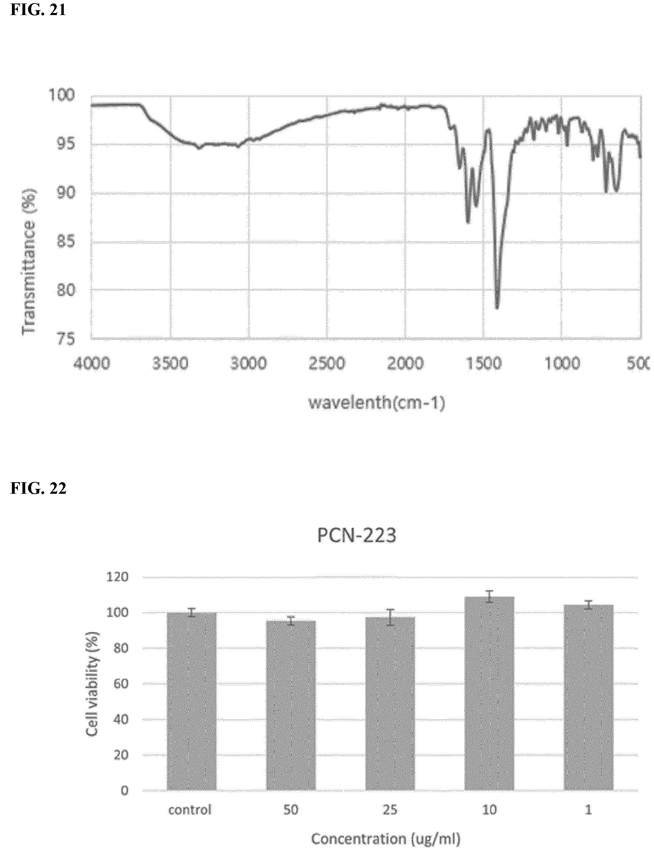

The zeta potential of MOF-525 was 14.51 mV (FIG. 20), and the results of FT-IR analysis of MOF-525 are shown in FIG. 21.

### Cytotoxicity Evaluation

L929 (fibroblasts) were seeded in a 96-well plate at a density of 9,000 cells per well and then incubated at 37°C for 24 hours. Suspensions with MOF concentrations of 50 µg/mL, 25 µg/mL, 10 µg/mL, and 1 µg/mL were prepared using cell culture medium. The existing medium of the cells in the 96-well plate cells was removed, and 100 µl of the suspension with each MOF concentration was added to each well, followed by incubation at 37°C for 24 hours. The suspension in the well was removed, and the suspension was washed out using PBS. 100 µL of medium containing 10% Ez-Cytox was added to each well. After incubation for about 1 hour, the absorbance was measured at 450 nm using a microplate reader (reference wavelength: 600 nm to 650 nm).

FIGS. 22 to 24 show the results of evaluating cytotoxicity.

FIG. 22 shows the results of evaluating the cytotoxicity of PCN-223, and it was confirmed that PCN-223 was not cytotoxic up to a MOF concentration of 50 µg/mL.

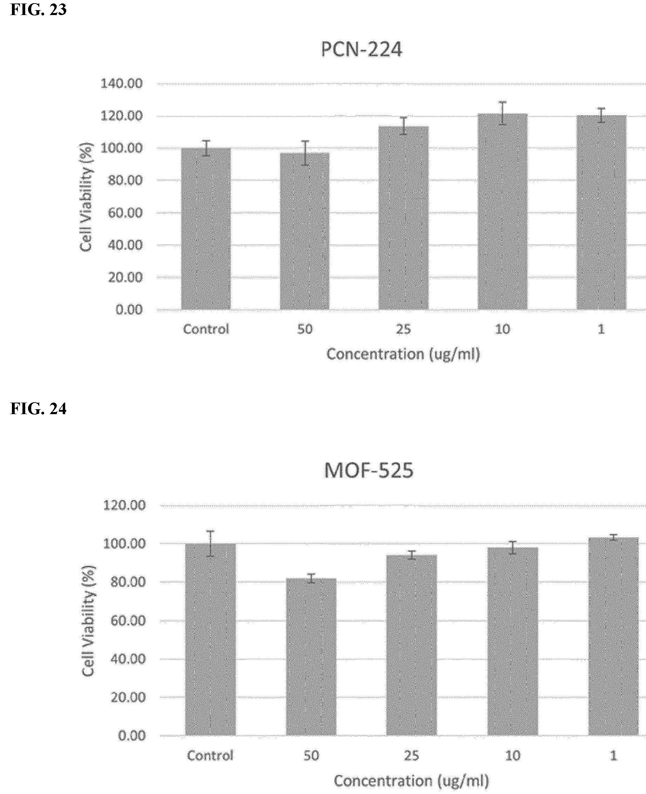

FIG. 23 shows the results of evaluating the cytotoxicity of PCN-224, and it was confirmed that PCN-224 was not cytotoxic up to a MOF concentration of 50 µg/mL.

FIG. 24 shows the results of evaluating the cytotoxicity of MOF-525, and it was confirmed that MOF-525 was not cytotoxic up to a MOF concentration of 50 µg/mL.

### Evaluation of Photodynamic Therapy

A PCN-223 suspension containing 50 µg/ml of PCN-223 was prepared using cell culture medium. 100 µl of the PCN-223 suspension was injected into a 96-well black plate. A 100 µM singlet oxygen sensor green (SOSG) reagent for detecting ROS activity was suspended in cell culture medium, thus preparing a 10 µM SOSG suspension. 100 µl of the 10 µM SOSG suspension was injected into the well containing the PCN-223 suspension, and then the two suspensions were well mixed by pipetting. A control well was injected with 100 µl of cell culture medium instead of the PCN-223 suspension and injected with 100 µl of the 10 µM SOSG suspension. Each well was irradiated with a laser at each of 100 mW, 200 mW, 300 mW, 400 mW, and 500 mW for 5 minutes. The amount of ROS released was evaluated by measuring the intensity of the SOSG reagent using a microplate reader.

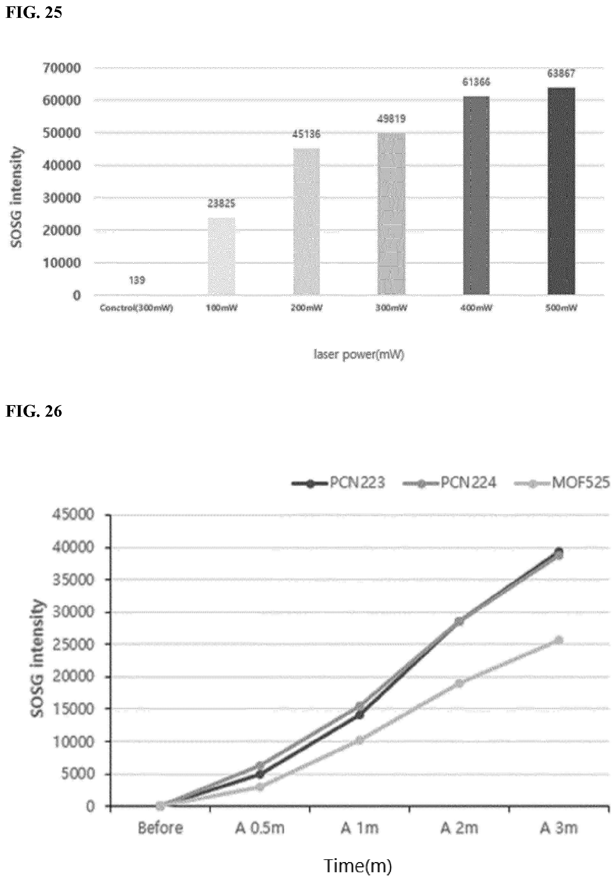

The test results are shown in FIG. 25. It was confirmed that the amount of ROS released increased as the laser power increased.

As an additional test, the same test as the previous test for evaluating photodynamic therapy was conducted. Here, the laser power was fixed at 500 mW, and laser irradiation was performed for 30 seconds, 30 seconds (cumulative time: 1 minute), 1 minute (cumulative time: 2 minutes), and 1 minute (cumulative time: 3 minutes). At each time point, the amount of ROS released was evaluated by measuring the intensity of the SOSG reagent using a microplate reader.

The test results are shown in FIG. 26. It can be seen that the amount of ROS released increased as the light irradiation time increased.

### Cytotoxicity Test against Colorectal Cancer Cells

MC38 (colorectal cancer cells) were seeded in a 96-well plate at a density of 9,000 cells per well and then incubated at 37°C for 24 hours. Each of PCN-223, PCN-224 and MOF-525 was suspended in cell culture medium at a concentration of 50 µg/ml, thus preparing PCN-223, PCN-224 and MOF-525 suspensions. The existing medium of the cells in the 96-well plate was removed, and 100 µl of each of the PCN-223, PCN-224 and MOF-525 suspensions with a concentration of 50 µg/ml was added to each well. However, two control wells were injected with 100 µl of cell culture medium instead of the suspension. Each well was irradiated with a laser at 500 mW for 5 minutes, followed by a rest period, and then irradiated once more with a laser for 5 minutes (cumulative time: 10 minutes). At this time, one of the control groups was not irradiated with a laser. The wells were incubated at 37°C for 24 hours. The suspensions in the wells were removed and the suspensions were washed out using PBS. 100 µL of medium containing 10% Ez-Cytox was added to each well. After incubation for 1 hour, absorbance was measured at 450 nm and 600 nm using a microplate reader (reference wavelength 600 nm), and cell viability was calculated according to the conversion formula.

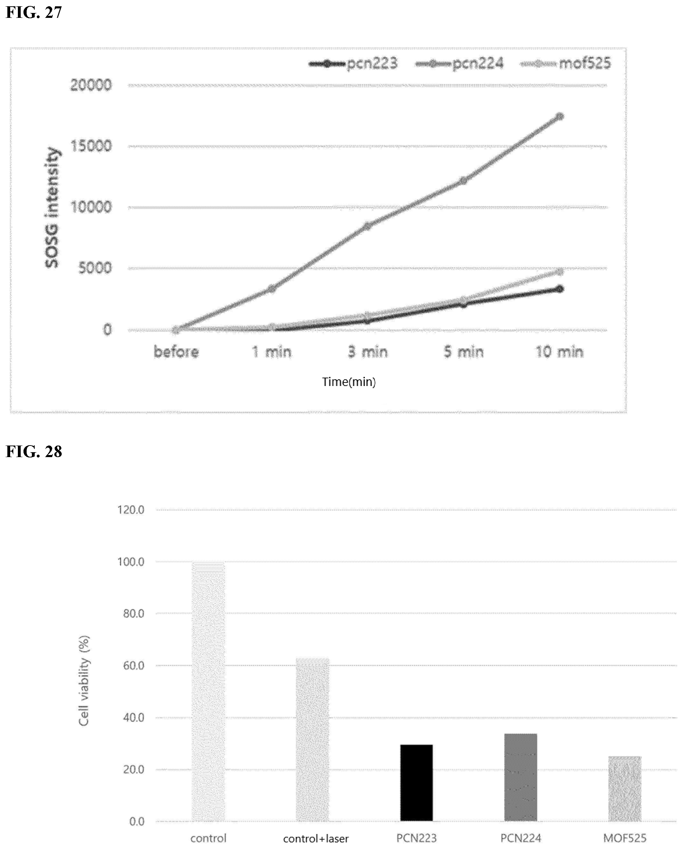

The test results are shown in FIG. 28.

It was confirmed that the effect of killing the colorectal cancer cell line was better in the groups treated with PCN-223, PCN-224 and MOF-525, respectively, than in the control group and the control group irradiated only with the laser.

### Production Example 2

### Production of Metal-Organic Framework Having Cancer Immunotherapeutic Agent Linked Thereto

PCN-223 particles were pretreated in a vacuum oven for 12 hours to obtain unsaturated metal sites. 5 mg of resiquimod was dissolved in 5 mL of ethanol, and then 50 mg of PCN-223 was added thereto, followed by stirring for 24 hours. After 24 hours, the particles were precipitated by centrifugation at 17,000 rpm for 10 minutes and the supernatant was removed. 10 ml of fresh ethanol was added thereto and the washing process was repeated three times. After final centrifugation, the sample was collected and dried in an oven at 80°C for 1 hour, thereby producing PCN-223 loaded with resiquimod (hereinafter referred to as PCN-223/resiquimod).

According to the same manner as the production method for PCN-223/resiquimod, PCN-224/resiquimod and MOF-525/resiquimod were produced using PCN-224 and MOF-525, respectively.

### Experimental Example 2

### Evaluation of Amount of Resiquimod Loaded

5 mg of PCN-223/resiquimod, PCN-224/resiquimod, or MOF-525/resiquimod was taken, added to 20 mL of deionized water, and then suspended using a sonicator, and 1 mL of 37% hydrochloric acid was added thereto. The mixture was placed in an oven at 37°C for 24 hours to disrupt the particles, and then 1 mL was sampled. The amount of resiquimod in the sample was measured using high performance liquid chromatography (HPLC) and converted to the amount of resiquimod loaded in the PCN-223/resiquimod, PCN-224/resiquimod, or MOF-525/resiquimod sample.

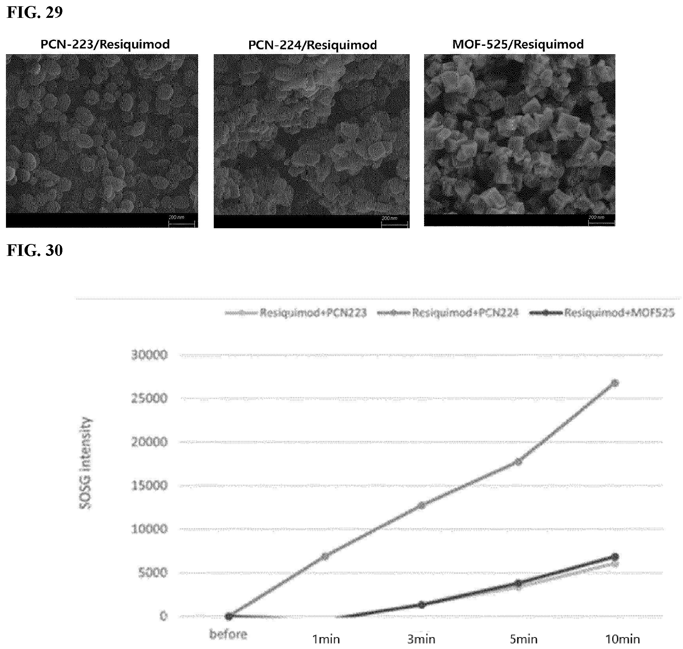

As a result of observing the morphology of PCN-223/resiquimod using a scanning electron microscope, the change in the size and morphology of the particles due to drug loading was not found (FIG. 29). In addition, as a result of analyzing the amount of resiquimod loaded in PCN-223/resiquimod by HPLC, it was shown that the amounts of resiquimod loaded in PCN-223/resiquimod, PCN-224/resiquimod, and MOF-525/resiquimod were 12.8 µg/mg, 9.3 µg/mg, and 13.1 µg/mg, respectively.

### Evaluation of Photodynamic Therapy

A PCN-223 suspension containing 50 µg/ml of PCN-223 was prepared using cell culture medium. 100 µl of the PCN-223 suspension was injected into a 96-well black plate. A 100 µM singlet oxygen sensor green (SOSG) reagent for detecting ROS activity was suspended in cell culture medium, thus preparing a 10 µM SOSG suspension. 100 µl of the 10 µM SOSG suspension was injected into the well containing the PCN-223 suspension, and then the two suspensions were well mixed by pipetting. A control well was injected with 100 µl of cell culture medium instead of the PCN-223 suspension and injected with 100 µl of the 10 µM SOSG suspension. Each well was irradiated with a laser at 500 mW for 1 minute, 2 minutes (cumulative time: 3 minutes), 2 minutes (cumulative time: 5 minutes), and 5 minutes (cumulative time: 10 minutes). The amount of ROS released was evaluated by measuring the intensity of the SOSG reagent using a microplate reader.

The test results are shown in FIG. 30. It was confirmed that the amount of ROS released increased as the laser irradiation time increased.

### Cytotoxicity Test against Colorectal Cancer Cells

CT26 (colorectal cancer cells) were seeded in a 96-well plate at a density of 9,000 cells per well and then incubated at 37°C for 24 hours. Each of PCN-223/resiquimod, PCN-224/resiquimod and MOF-525/resiquimod was suspended in cell culture medium at a concentration of 50 µg/ml, thus preparing PCN-223/resiquimod, PCN-224/resiquimod and MOF-525/resiquimod suspensions. The existing medium of the cells in the 96-well plate was removed, and 100 µl of each of the PCN-223/resiquimod, PCN-224/resiquimod and MOF-525/resiquimod suspensions with a concentration of 50 µg/ml was added to each well. However, two control wells were injected with 100 µl of cell culture medium instead of the suspension. Each well was irradiated with a laser at 500 mW for 5 minutes, followed by a rest period, and then irradiated once more with a laser for 5 minutes (cumulative time: 10 minutes) At this time, one of the control groups was not irradiated with a laser. The wells were incubated at 37°C for 24 hours. The suspensions in the wells were removed and the suspensions were washed out using PBS. 100 µL of medium containing 10% Ez-Cytox was added to each well. After incubation for about 1 hour, absorbance was measured at 450 nm using a microplate reader (reference wavelength 600 nm), and cell viability was calculated according to the conversion formula.

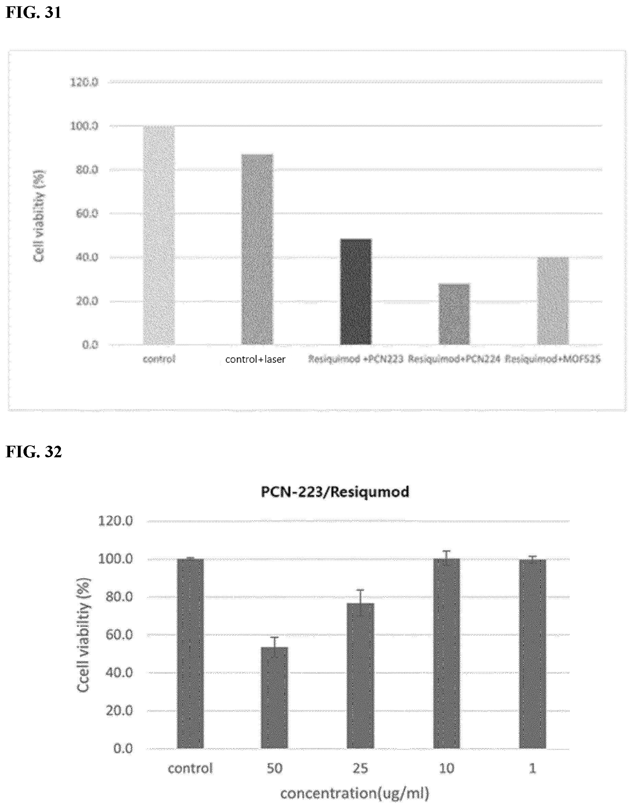

The test results are shown in FIG. 31. It can be seen that, in the medium treated with each of the PCN-223/resiquimod, PCN-224/resiquimod and MOF-525/resiquimod of the present invention, the cell viability of CT26 was rapidly reduced by laser irradiation. This is because each of PCN-223/resiquimod, PCN-224/resiquimod and MOF-525/resiquimod exhibited a photodynamic therapeutic effect and the effect of releasing resiquimod, upon light irradiation.

### Test for Cytotoxicity of PCN-223/resiquimod

L929 cells (murine fibroblast cells) were incubated in a cell incubator at 37°C under 5% CO₂, and then seeded in a 96-well plate at 1×10⁴ cells/well. After incubation for 24 hours, the medium in each well was removed, and each of PCN-223/resiquimod, PCN-224/resiquimod, and MOF-525/resiquimod was suspended in fresh medium at concentrations of 1 µg/ml, 10 µg/ml, 25 µg/ml, and 50 µg/ml, and each suspension was added at a volume of 100 µl per well. After incubation at 37°C under 5% CO₂ for 24 hours, the particle suspensions in the plate were removed, and each well was washed three times with DPBS. Finally, 90 µl of fresh medium and 10 µl of EZ-cytox reagent were added to each well, and then each well was incubated in a cell incubator at 37°C for 1 hour. Then, the cell viability was determined by measuring the absorbance at 450 nm and 600 nm using a microplate reader.

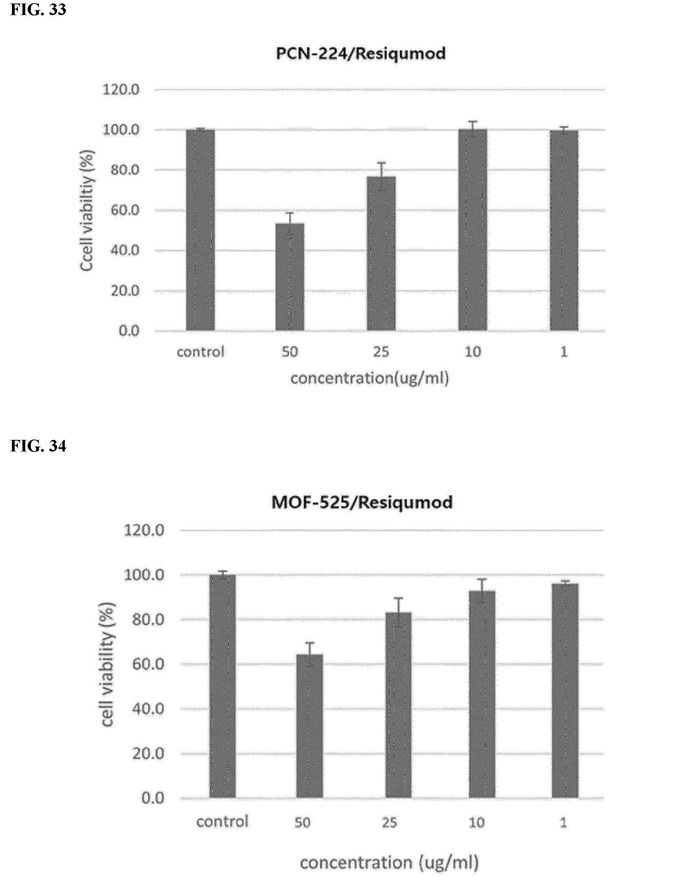

The results of evaluating the cytotoxicity of PCN-223/resiquimod, PCN-224/resiquimod, and MOF-525/resiquimod are shown in FIGS. 32, 33 and 34, respectively.

It was confirmed that the groups treated with PCN-223/resiquimod, PCN-224/resiquimod, and MOF-525/resiquimod, respectively, exhibited a cell viability of 100% or higher at concentrations of 1 µg/ml and 10 µg/ml included in the test concentration range, indicating that PCN-223/resiquimod, PCN-224/resiquimod, and MOF-525/resiquimod were not cytotoxic. However, it was confirmed that treatment at a concentration of 25 µg/ml or higher exhibited cytotoxicity, but this cytotoxicity was due to resiquimod linked to each of PCN-223, PCN-224, and MOF-525.

Although the preferred embodiments of the present invention have been described in detail above, the scope of the present invention is not limited thereto, and various modifications and improvements made by those skilled in the art using the basic concept of the present invention as defined in the following claims also fall within the scope of the present invention.

### Industrial Applicability

The present invention relates to an anticancer composition comprising a metal-organic framework (MOF) having a cancer immunotherapeutic agent linked thereto and the use thereof.

## Claims

1. An anticancer composition comprising a metal-organic framework (MOF) having a cancer immunotherapeutic agent linked thereto.

2. The anticancer composition according to claim 1, wherein the metal-organic framework comprises a metal cluster and a ligand compound represented by Formula 1 below that coordinates to the metal cluster: 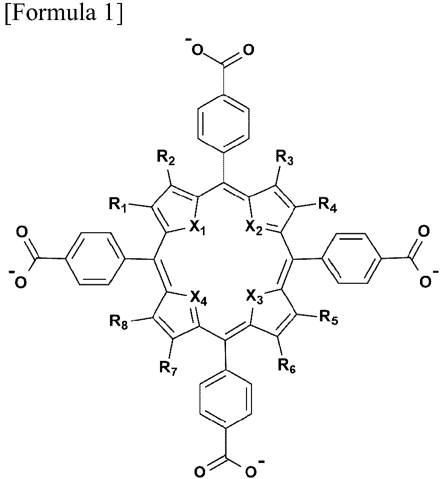 wherein
X₁ and X₃ are each independently N(R₉),
X₂ and X₄ are each independently N, and
R₁ to R₉ are the same as or different from one another and are each independently selected from the group consisting of hydrogen, deuterium, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

3. The anticancer composition according to claim 1, wherein the metal-organic framework comprises either a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir³⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ and Bi⁺.

4. The anticancer composition according to claim 3, wherein the metal-organic framework is selected from the group consisting of an aluminum-based metal-organic framework, an iron-based metal-organic framework, a zirconium-based metal-organic framework, and mixtures thereof.

5. The anticancer composition according to claim 2, wherein the cancer immunotherapeutic agent coordinates to an unsaturated metal site of the metal cluster.

6. The anticancer composition according to claim 1, wherein the cancer immunotherapeutic agent is an immune-modulating agent.

7. The anticancer composition according to claim 6, wherein the immune-modulating agent is an agonist of TLR7 (Toll-like receptor 7) or TLR8.

8. The anticancer composition according to claim 1, wherein the cancer is colorectal cancer, liver cancer, lung cancer, breast cancer, melanoma, gastric cancer, colon cancer, skin cancer, ovarian cancer, cervical cancer, thyroid cancer, kidney cancer, prostate cancer, bladder cancer, pancreatic cancer, esophageal cancer, or fibrosarcoma.

9. The anticancer composition according to claim 1, wherein the metal-organic framework generates singlet oxygen upon light irradiation.

10. A method for treating cancer, comprising a step of administering the anticancer composition according to claim 1 to a subject in an amount effective for treating the cancer and irradiating the subject with light.
